# EUROPÄISCHE PATENTSCHRIFT

(11) **EP 1 274 672 B1**
(45) Veröffentlichungstag und Bekanntmachung des Hinweises auf die Patenterteilung: **03.03.2004**
(21) Anmeldenummer: 01921367.7
(22) Anmeldetag: 30.03.2001
(51) Int. Cl.: C07C 201/12, C07C 205/57, C07C 205/58, C07C 303/22

(54) **VERFAHREN ZUR HERSTELLUNG VON SUBSTITUIERTEN NITRO-BENZOESÄUREN DURCH OXIDATION DER KORRESPONDIERENDEN NITRO-TOLUOLE, NITRO-BENZYLALKOHOLE, -ESTER UND/ODER -ETHER**
METHOD FOR PRODUCING SUBSTITUTED NITRO BENZOIC ACIDS BY OXIDATION OF CORRESPONDING NITRO TOLUENES, NITRO BENZYL ALCOHOLS, ESTERS AND/OR ETHERS
PROCEDE DE PRODUCTION D'ACIDES NITRO-BENZOIQUES PAR OXYDATION DE NITRO-TOLUENES, D'ALCOOLS NITRO-BENZYLIQUES, DE LEURS ESTERS ET/OU ETHERS CORRESPONDANTS

(30) Priorität: 12.04.2000 DE 10018048
(43) Veröffentlichungstag der Anmeldung: 15.01.2003
(73) Patentinhaber: Bayer Chemicals AG, 51368 Leverkusen (DE)
(72) Erfinder: LANGER, Reinhard, 47918 Tönisvorst (DE); RODEFELD, Lars, 51371 Leverkusen (DE); NEUMANN, Karl-Heinz, 53757 St. Augustin (DE)
(86) Internationale Anmeldenummer: PCT/EP2001/003667
(87) Internationale Veröffentlichungsnummer: WO 2001/079151

(56) Entgegenhaltungen:
- EP-A- 0 916 653
- DE-A- 19 927 408
- GB-A- 705 195
- GB-A- 960 246
- GB-A- 1 068 535

## Beschreibung

Die vorliegende Erfindung betrifft ein Verfahren zur Herstellung von speziell substituierten Nitro-benzoesäuren durch Oxidation der korrespondierenden Nitrotoluole, Nitrobenzylalkohole, deren Ester und/oder Ether mit Salpetersäure.

Substituierte Nitro-benzoesäuren stellen interessante Zwischenprodukte für pharmazeutische und agrochemische Wirkstoffe dar.

Nitrotoluole mit zusätzlichen elektronenziehenden, speziell sauerstoffhaltigen Substituenten sind Verbindungen, die beim autothermen Zerfall zu einer erheblichen Energiefreisetzung führen. Die Oxidation solcher Verbindungen ist daher angesichts der gleichzeitig erforderlichen hohen Reaktionstemperaturen sicherheitstechnisch schwierig zu realisieren. Sie bedarf bei einer Durchführung im industriellen, großtechnischen Maßstab einer erheblichen Kontrolle, und es müssen besondere Maßnahmen zur Minimierung des sicherheitstechnischen Risikos getroffen werden.

Kommerziell werden elektronenarme Nitrotoluole mit Salpetersäure in kontinuierlich betriebenen Verweilzeitrohren oxidiert, wie dies in der Chemiker-Zeitung 104 (1980), S. 349-351 beschrieben wird. Ein Gemisch aus Salpetersäure und Toluol wird vorgeheizt und unter hohem Druck durch ein senkrecht stehendes Reaktorrohr gepumpt. Es wird darauf hingewiesen, dass hierbei das Risiko einer Explosion immer gegeben ist, weshalb ein Schutzwall zwischen Reaktor und Kontrollraum aufgebaut wird und die Reaktion nur per Femsteuerung aus dem Kontrollraum heraus durchgeführt werden kann.

Weiterer Nachteil einer derartigen kontinuierlichen Prozessführung ist, dass durch Anund Abfahrzustände nicht spezifikationsgerechte Ware entstehen kann, was speziell bei Produkte mit kleineren Tonnagen problematisch ist.

GB-A-705 195 beschreibt die Oxidation von Toluolsulfonsäuren und Nitro-toluol-sulfonsäuren mit Salpetersäure bei 120 - 300°C im Autoklaven, gegebenenfalls unter Zuhilfenahme von Sauerstoff, nitrosen Gasen, Metallnitraten oder Metallnitriten. Die Komponenten werden zusammengegeben und der Druckreaktor erhitzt, bis eine stark exotherme Reaktion eintritt. Beschrieben wird, dass die Umsetzung sowohl kontinuierlich als auch semikontinuierlich durchgeführt werden kann. Bei der semikontinuierlichen Vorgehensweise wird das Oxidationsmittel zu der (Nitro)Toluolsulfonsäure zudosiert. Ähnliche Verfahrensweisen werden in GB-A-705 195, GB-A-960 246 und DE-A-1 068 535, für andere Edukte, offenbart.

DE-A-197 49 723 beschreibt die Herstellung von 4-Nitro-2-sulfo-benzoesäure durch Oxidation von 4-Nitro-toluol-2-sulfonsäure bei 120 - 170°C und einem Druck von bis zu 12 bar, wobei das Toluol gelöst in Wasser vorgelegt und die Salpetersäure zudosiert wird. Auf die sicherheitstechnischen Belange dieser Umsetzung wird explizit hingewiesen. Hinzukommt, dass die in diesem Verfahren erzielbare Ausbeute von 75-85% im Hinblick auf eine industrielle Herstellung nicht zufriedenstellend ist. Ein ähnliches Verfahren wird in EP-A-0 916 653 offenbart.

Die Aufgabe der vorliegenden Erfindung bestand somit darin, ein Oxidationsverfahren bereitzustellen, mit dem substituierte Nitro-benzoesäuren ohne sicherheitstechnisches Risiko und mit hohen reproduzierbaren Ausbeuten auch in kleinen Tonnagen erhalten werden können.

Überraschenderweise wurde gefunden, dass diese Aufgabe gelöst werden kann, wenn in Rahmen eines Semibatch-Verfahrens zunächst das Oxidationsmittel vorgelegt wird und anschließend bei erhöhter Temperatur und erhöhtem Druck das Edukt in Form eines Nitro-toluols, Nitro-benzylalkohols, -esters und/oder -ethers zudosiert wird.

Gegenstand der Erfindung ist ein Verfahren zur Herstellung von Nitro-benzoesäuren der allgemeinen Formel (I) 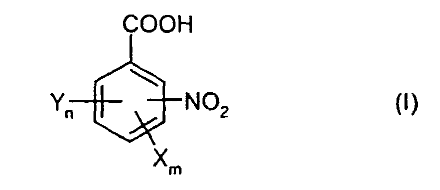 worin
- X: unabhängig voneinander Nitro, Cyano, Sulfo, Carboxy, Carbonylphenyl, Trifluormethyl oder Trichlormethyl bedeuten,
- Y: unabhängig voneinander Phenyl, Fluor, Chlor, Brom oder Jod bedeuten und in denen der Phenylrest wiederum mit bis zu 3 Resten X und/oder Y ungleich Wasserstoff substituiert sein kann,
- m: den Wert 0, 1, 2 oder 3 und
- n: den Wert 0, 1, 2 oder 3 besitzt,
und die Summe aus m und n den Wert 0, 1, 2, 3 oder 4 besitzt,
aus Verbindungen der allgemeinen Formel (II) 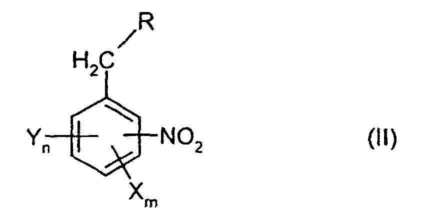 worin
- X, Y, m, n: sowie die Summe aus n und m die für die allgemeine Formel (I) genannten Bedeutungen haben und
- R: für H oder einen Rest OR' steht und
- R': für H, geradkettiges oder verzweigtes C₁-C₁₀-Alkyl oder geradkettiges oder verzweigtes C₁-C₁₀-Carbonylalkyl steht oder R' einen Rest der allgemeinen Formel (III) 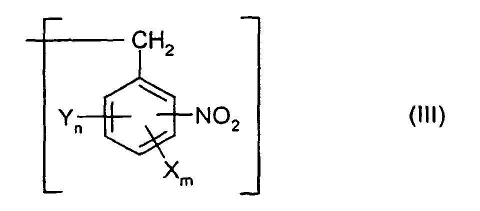 oder einen Rest der allgemeinen Formel (IV) darstellt, 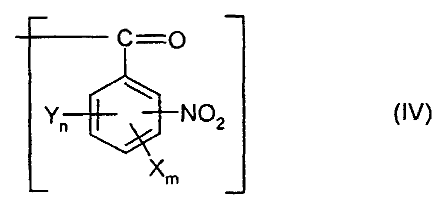 worin X, Y, n, m sowie die Summe aus n und m jeweils die für die allgemeine Formel (I) genannten Bedeutungen besitzen,
dadurch gekennzeichnet, dass ein Gemisch aus Salpetersäure und Wasser bei einem Druck im Bereich von 2-20 bar auf Temperaturen im Bereich von 100 - 220°C erhitzt wird und in dieses erhitzte Gemisch aus Salpetersäure und Wasser die Verbindung der allgemeinen Formel (II) dosiert wird,
wobei jedoch als Verbindungen der allgemeinen Formel (II) 2-Halogen-6-nitrobenzylalkohole, -ester oder -ether der allgemeinen Formel (V) ausgenommen sind, 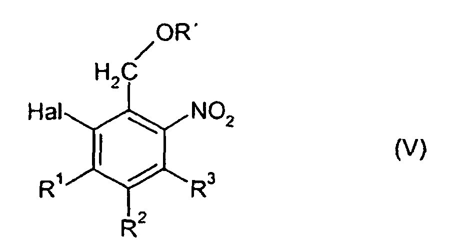 worin
- R': die für die allgemeine Formel (II) genannte Bedeutung hat,
- R¹, R² und R³: unabhängig voneinander H, Fluor, Chlor, Brom, Nitro oder Carboxyl bedeuten und
- Hal: für Fluor, Chlor oder Brom steht.

Ein entscheidender Faktor des erfindungsgemäßen Verfahrens ist die Art der Vermischung des Eduktes und der Salpetersäure dergestalt, dass die Salpetersäure im Gemisch mit Wasser vorgelegt und erhitzt wird und das zu oxidierende Edukt anschließend zudosiert wird. Hierdurch wird die stationäre Konzentration des Eduktes, dessen Zersetzungstemperatur häufig nah an dem Bereich liegt, in dem die Wärmeentwicklung der gewünschten Reaktion stattfindet, im Reaktionsgemisch besonders niedrig gehalten. Sobald das Edukt in das Salpetersäure-Reaktionsgemisch gelangt, reagiert es sofort mit der Salpetersäure zu unreaktiverem Produkt. Die Edukt-Konzentrationen im Reaktionsgemisch liegen im erfindungsgemäßen Verfahren bei maximal 7 Gew.-%, bevorzugt bei maximal 6 Gew.-% und insbesondere maximal bei 5 Gew.-%. Auf diese Art und Weise wird das Gefährdungspotential der Oxidation ganz maßgeblich verringert.

In den allgemeinen Formeln (I) und (II) steht X unabhängig voneinander bevorzugt für einen Sulfonsäure- oder einen Nitrorest und Y unabhängig voneinander bevorzugt für Fluor oder Chlor. Der Index n steht bevorzugt für einen Wert 0, 1 oder 2 und der Index m steht ebenfalls bevorzugt für einen Wert 0, 1 oder 2, wobei die Summe aus n und m bevorzugt für den Wert 1 oder 2 steht. Bevorzugt befinden sich in einer der o-Positionen zu der zu oxidierenden Gruppe -CH₂R ein Wasserstoff-Atom oder ein Fluoratom, bevorzugt ein Wasserstoffatom.

Das erfindungsgemäße Verfahren wird bevorzugt auf solche Verbindungen der allgemeinen Formel (II) angewendet, die eine ausgeprägte Neigung zur autothermen Zersetzung aufweisen. Besonders bevorzugt werden Nitrotoluole und insbesondere Nitro-toluol-sulfonsäuren als Edukte eingesetzt. Ganz besonders bevorzugt wird 4-Nitro-toluol-2-sulfonsäure zu 4-Nitro-2-sulfo-benzoesäure oxidiert.

Das erfindungsgemäße Verfahren wird bei einem Druck von 2 - 20 bar, bevorzugt von 4 - 15 bar, besonders bevorzugt von 6 - 12 bar und insbesondere von 10-12 bar durchgeführt. Da im Reaktionsverlauf Stickoxide freiwerden, ist es sinnvoll, den Druck über ein Druckhalteventil zu kontrollieren und die Stickoxide im erforderlichen Umfang darüber entweichen zu lassen.

Die Temperatur, bei der das erfindungsgemäße Verfahren durchgeführt wird, liegt im Bereich von 100 - 220°C, bevorzugt von 120 - 200°C und besonders bevorzugt von 140 - 180°C.

Für die Umsetzung wird eine 10-90%ige und bevorzugt eine 20-65%ige Salpetersäure vorgelegt.

Das molare Verhältnis der Verbindung der allgemeinen Formel (II) zur Salpetersäure liegt im Bereich von (2-20):1, bevorzugt im Bereich von (3-10):1 und besonders bevorzugt im Bereich von (4-8):1.

Das erfindungsgemäße Verfahren wird üblicherweise derart durchgeführt, dass die Salpetersäure in einem Druckreaktor vorgelegt und aufgeheizt wird. Danach wird die Eduktlösung oder Schmelze innerhalb von 1 - 48, bevorzugt von 2 - 24 und besonders bevorzugt von 4 - 12 Stunden zudosiert. An die erfolgte Zudosierung schließt sich gegebenenfalls noch eine gewisse Nachreaktionszeit bei der Reaktionstemperatur zwecks Komplettieren des Umsatzes an. Diese Nachreaktionszeit liegt üblicherweise im Bereich von 1-15 Stunden und bevorzugt im Bereich von 3-8 Stunden.

Nach beendeter Oxidation und gegebenenfalls dem Ablauf der Nachrührzeit kann man das Reaktionsgemisch abkühlen, das Reaktionsgefäß entspannen und restliche Stickoxide ausblasen, z.B. mit Stickstoff.

Die Isolierung von erfindungsgemäß hergestellten Nitro-sulfo-benzoesäuren der allgemeinen Formel (I) kann beispielsweise durch Zugabe einer wässrigen, vorzugsweise gesättigten Kaliumchlorid-Lösung und Filtration des dann ausfallenden Monohydrats des Kaliumsalzes der Nitro-sulfo-benzoesäure erfolgen. Nach dessen Trocknung erhält man im allgemeinen ein Produkt mit einer Reinheit von über 95 Gew.-% (bestimmt durch HPLC).

Man kann die Fällung des Kaliumsalzes auch so vornehmen, dass man eine wässrige Kaliumhydroxidlösung, beispielsweise mit einer Konzentration von 30-50 Gew.-%, zugibt, bis der pH-Wert im Bereich von beispielsweise 0,5-2,5 liegt. Der größte Teil der Nitro-sulfo-benzoesäure fällt dann als Kaliumsalz aus. Gegebenenfalls kann man die Fällung durch Zugabe einer wässrigen, vorzugsweise gesättigten Kaliumchlorid-Lösung vervollständigen.

Sonstige Methoden zur Isolierung der durch Oxidation erhaltenen Benzoesäurederivate der allgemeinen Formel (I) sind dem Fachmann im allgemeinen bekannt.

In einer besonderen Ausführungsform des erfindungsgemäßen Verfahrens werden 10-90%, bevorzugt 20-50% der einzusetzenden Salpetersäure vorgelegt und aufgeheizt, und dann werden simultan aber getrennt das Edukt und die restliche Salpetersäure in den Reaktor eindosiert.

Weiterhin kann es von Vorteil sein, zur vorgelegten Salpetersäure Substanzen hinzuzufügen, die NOₓ freisetzen oder beinhalten, wie z.B. Alkali- oder Erdalkalinitrite, salpetrige Säure oder konzentrierte rotbraune Salpetersäure. Hierdurch kann man die benötigte Reaktionstemperatur herabsetzen und damit das Gefahrenpotential weiter vermindern.

Das Edukt der allgemeinen Formel (II) kann entweder als Schmelze oder gelöst in einem Lösungsmittel in das Salpetersäure-Reaktionsgemisch zudosiert werden. Als Lösungsmittel sind schwer bzw. nicht oxidierbare Flüssigkeiten geeignet, die unter den gegebenen Reaktionsbedingungen inert gegenüber Salpetersäure sind, bevorzugt Wasser, Nitrobenzol, 1,2,4-Trichlorbenzol oder Methylenchlorid. Leichtsiedende Lösungsmittel wie Methylenchlorid werden mit dem NOₓ-Strom aus dem Reaktor transportiert.

Bevorzugt werden im erfindungsgemäßen Verfahren die Verbindungen der allgemeinen Formel (II) und insbesondere die Nitro-sulfo-toluole als 20-70%ige, bevorzugt als 30-55%ige wässrige Lösung in die Salpetersäure zudosiert.

DTA-Untersuchungen der Reaktionsgemische des erfindungsgemäßen Verfahrens zur Oxidation der Nitro-verbindungen der allgemeinen Formel (II) ergaben keine Anhaltspunkte für ein sicherheitstechnisches Gefährdungspotential; geringfügig exotherme Reaktionen werden im Fall der vorliegenden Reaktionsgemische erst bei Temperaturen im Bereich von 260-370°C beobachtet. Dies ist ein sicherheitstechnisch erheblicher Vorteil gegenüber dem nächstliegenden Verfahren des Standes der Technik mit umgekehrter Durchführung, d.h. der Vorlage von z.B. 4-Nitro-toluol-sulfonsäure und anschließender Zudosierung von Salpetersäure. DTA-Untersuchungen solcher Reaktionsgemische zeigen, dass erste exotherme Zersetzungsreaktionen bereits bei 175°C einsetzen und oberhalb von 250°C die weitere Zersetzung ganz massiv verläuft.

### Beispiele:

### Beispiel 1 (Vergleich)

In einem 11 Tantalautoklaven mit Rührer, Rückflußkühler und einem aufgesetztem und auf 5 bar eingestelltem Druckhalteventil werden 108,6 g wasserfeuchte 4-Nitrotoluol-2-sulfonsäure (0,39 mol) in 120 g Wasser suspendiert und auf 140 °C erhitzt. Dabei baut sich ein Druck von ca. 3,6 bar auf. Nach Erreichen dieser Temperatur werden 180 g 70 Gew.-%ige Salpetersäure innerhalb von 5 h in den Autoklaven eingepumpt. Der Druck steigt auf Grund der NOₓ-Entwicklung bis auf die vorgegebenen 5 bar an, nach Überschreiten dieses Druckes werden die entstandenen Stickoxide abgeblasen. Nach beendeter Salpetersäuredosierung wird noch 12 h bei 140 °C nachgerührt. In den letzten Stunden der Nachrührzeit sinkt der Druck wieder unter 5 bar ab und der Abgasstrom versiegt. Nachdem der Autoklav auf Raumtemperatur abgekühlt ist, wird entspannt und die restlichen Stickoxide mit Stickstoff verdrängt. Der Autoklaveninhalt wird in einen Kolben mit Rührer überführt, und es werden 150 g einer kalt gesättigten wässrigen KCl-Lösung zugegeben. Die 4-Nitro-2-sulfobenzoesäure fällt als Kaliumsalz aus, wird abfiltriert, mit kaltem Wasser gewaschen und im Trockenschrank getrocknet.

Es werden 96 g Kaliumsalz-monohydrat der 4-Nitro-2-sulfobenzoesäure mit einem Gehalt von 97 % (HPLC) isoliert, entsprechend einer Ausbeute von 78,5% bezogen auf die eingesetzte 4-Nitro-toluol-2-sulfonsäure.

In Tabelle 1 ist die Edukt-Konzentration im Reaktionsgemisch in Abhängigkeit von der Reaktionszeit dargestellt.

### Beispiel 2 (Vergleich)

In der in Beispiel 1 beschriebenen Apparatur werden 108,6 g wasserfeuchte 4-Nitrotoluol-2-sulfonsäure (0,39 mol) in 120 g Wasser suspendiert und auf 160 °C erhitzt.

Dabei baut sich ein Druck von ca. 6,1 bar auf. Nach Erreichen der Temperatur werden 180 g 70 gew.%ige Salpetersäure innerhalb von 4 h in den Autoklaven eingepumpt. Der Druck steigt auf Grund der NOₓ-Entwicklung bis auf die vorgegebenen 9 bar an, nach Überschreiten diesen Druckes werden die entstandenen Stickoxide abgeblasen. Nach beendeter Salpetersäuredosierung wird noch 1 h bei 160 °C nachgerührt. In den letzten Stunden der Nachrührzeit sinkt der Druck wieder unter 9 bar ab und der Abgasstrom versiegt.

Die Aufarbeitung erfolgt analog zu Beispiel 1, und es werden 92,4 g Kaliumsalz-monohydrat der 4-Nitro-2-sulfobenzoesäure mit einem Gehalt von 97 % (HPLC) isoliert, entsprechend einer Ausbeute von 75,6%, bezogen auf die eingesetzte 4-Nitro-toluol-2-sulfonsäure.

In Tabelle 1 ist die Edukt-Konzentration im Reaktionsgemisch in Abhängigkeit von der Reaktionszeit dargestellt.

### Beispiel 3

581,5 g 65%ige Salpetersäure werden in einem Stahl-Email-Autoklav auf 160°C erhitzt. Dann werden 684,8 g einer 31,7%igen 4-Nitrotoluol-2-sulfonsäure-Lösung über 10 Stunden zudosiert. Der Innendruck wird auf 12 bar gehalten. Anschließend läßt man 6 h bei dieser Temperatur nachrühren. Der Ansatz wird mit 150 g Wasser in eine Vorlage überführt, und man läßt über 2h bei 60 °C 389,5 g einer 50%igen Kaliumhydroxid-Lösung zutropfen. Nach 2 stündigem Nachrühren wird der Ansatz filtriert. Der Niederschlag wird anschließend zweimal mit je zur Hälfte 150 g EisWasser gewaschen. Das Produkt wird bei 50-80°C und 50-100 mbar getrocknet, wodurch 273,8 g Produkt erhalten werden, welches 99,1 %ig an 4-Nitro-2-sulfobenzoesäure Mono-Kaliumsalz und 0,3%ig an Edukt ist, bei einem Gehalt an Wasser und Kaliumnitrat von in der Summe ca. 0,6 %. Die Ausbeute beträgt somit 94 % der Theorie, bezogen auf die eingesetzte 4-Nitrotoluol-2-sulfonsäure.

In Tabelle 1 ist die Edukt-Konzentration im Reaktionsgemisch in Abhängigkeit von der Reaktionszeit dargestellt.

### Beispiel 4

581,5 g 65%ige Salpetersäure werden in einem Stahl-Email-Autoklav auf 140°C erhitzt. Dann werden 684,8 g einer 31,7%igen 4-Nitrotoluol-2-sulfonsäure-Lösung über 10 Stunden zudosiert. Der Innendruck wird auf 12 bar gehalten. Anschließend läßt man 6 h bei dieser Temperatur nachrühren.

Die Aufarbeitung des Reaktionsgemisches erfolgt wie in Beispiel 3 beschrieben. Man erhält ein Produkt, dass einen Restgehalt von 2% an 4-Nitrotoluol-2-sulfonsäure-Kaliumsalz enthält. Die Ausbeute an 4-Nitro-2-sulfobenzoesäure Mono-Kaliumsalz beträgt ca. 91 % der Theorie, bezogen auf die eingesetzte 4-Nitrotoluol-2-sulfonsäure.

In Tabelle 1 ist die Edukt-Konzentration im Reaktionsgemisch in Abhängigkeit von der Reaktionszeit dargestellt. Die etwas niedrigere Temperatur als in Beispiel 3 führt zu stationären Konzentrationen des Eduktes unterhalb von 5 Gew.-%. Dieser Wert kann verringert werden, indem man der Salpetersäure vor der Reaktion 0,01 mol Natriumnitrit zufügt (Beispiel 5).

### Beispiel 5

581,5 g 65%ige Salpetersäure werden mit 690 mg (0,01 mol) Natriumnitrit versetzt und in einem Stahl-Email-Autoklav auf 140°C erhitzt. Dann werden 684,8 g einer 31,7%igen 4-Nitrotoluol-2-sulfonsäure-Lösung über 10 Stunden zudosiert. Der Innendruck wird auf 12 bar gehalten. Anschließend läßt man 6 h bei dieser Temperatur nachrühren.

Die Aufarbeitung des Reaktionsgemisches erfolgt wie in Beispiel 3 beschrieben, und man erhält ein Produkt, dass noch einen Restgehalt von 0,9 % 4-Nitro-toluol-sulfonsäure-Kaliumsalz enthält. Die Ausbeute an 4-Nitro-2-sulfobenzoesäure Mono-Kaliumsalz beträgt ca. 93% der Theorie, bezogen auf die eingesetzte 4-Nitrotoluol-2-sulfonsäure.

In Tabelle 1 ist die Edukt-Konzentration im Reaktionsgemisch in Abhängigkeit von der Reaktionszeit dargestellt. Die stationären Konzentrationen des Eduktes während der Reaktion überschreitet zu keinem Zeitpunkt 3 %.

**Tabellel:**

| Edukt-Konzentration im Reaktionsgemisch in Abhängigkeit von der Reaktionszeit | | |
|---|---|---|
| Beispiel | Zeit [Stunden] | Gehalt Edukt in Lösung [Gew.- %] |
| I (Vergleich) | 0 | 37 |
| | 2,5 | 13 |
| | 5 | 5 |
| | 17 | 0,7 |
| 2 (Vergleich) | 0 | 37 |
| | 2 | 11 |
| | 4 | 3 |
| | 5 | 0,8 |
| 3 | 3,3 | 0,48 |
| | 6,7 | 1,31 |
| | 10 | 2,11 |
| | 14 | 0,14 |
| 4 | 2 | 4,65 |
| | 4 | 3,69 |
| | 6 | 3 |
| | 8 | 3,5 |
| | 10 | 3,6 |
| | 16 | 0,64 |
| 5 | 2 | 2,62 |
| | 4 | 2,06 |
| | 6 | 2,38 |
| | 8 | 2,55 |
| | 10 | 2,83 |
| | 16 | 0,22 |

## Patentansprüche

1. Verfahren zur Herstellung von Nitro-benzoesäuren der allgemeinen Formel (I) 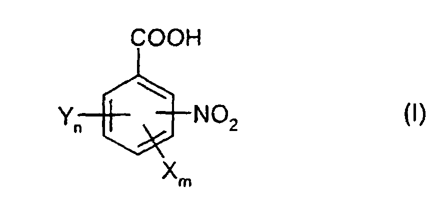 worin
X unabhängig voneinander Nitro, Cyano, Sulfo, Carboxy, Carbonylphenyl, Trifluormethyl oder Trichlormethyl bedeuten,
Y unabhängig voneinander Phenyl, Fluor, Chlor, Brom oder Jod bedeuten und in denen der Phenylrest wiederum mit bis zu 3 Resten X und/oder Y ungleich Wasserstoff substituiert sein kann,
m den Wert 0, 1, 2 oder 3 und
n den Wert 0, 1, 2 oder 3 besitzt,
und die Summe aus m und n den Wert 0, 1, 2, 3 oder 4 besitzt,
aus Verbindungen der allgemeinen Formel (II) 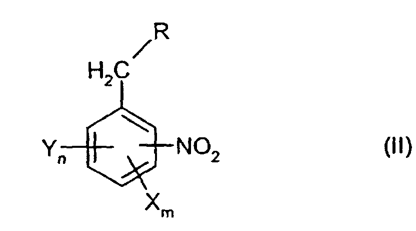 worin
X, Y, m, n sowie die Summe aus n und m die für die allgemeine Formel (I) genannten Bedeutungen haben und
R für H oder einen Rest OR' steht und
R' für H, geradkettiges oder verzweigtes C₁-C₁₀-Alkyl oder geradkettiges oder verzweigtes C₁-C₁₀-Carbonylalkyl steht oder R' einen Rest der allgemeinen Formel (III) 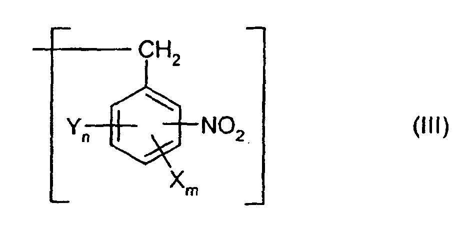
oder einen Rest der allgemeinen Formel (IV) darstellt, 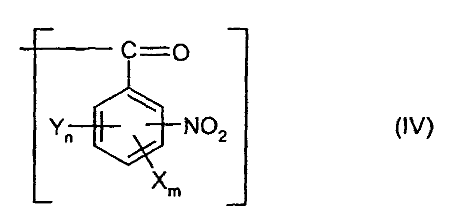 worin X, Y, n, m sowie die Summe aus n und m jeweils die für die allgemeine Formel (I) genannten Bedeutungen besitzen,
**dadurch gekennzeichnet, dass** ein Gemisch aus Salpetersäure und Wasser bei einem Druck im Bereich von 2-20 bar auf Temperaturen im Bereich von 100 - 220°C erhitzt wird und in dieses erhitzte Gemisch aus Salpetersäure und Wasser die Verbindung der allgemeinen Formel (II) dosiert wird,
wobei jedoch als Verbindungen der allgemeinen Formel (II) 2-Halogen-6-nitrobenzylalkohole, -ester oder -ether der allgemeinen Formel (V) ausgenommen sind, 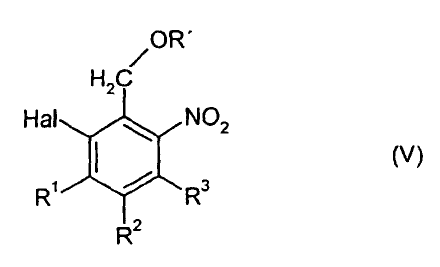 worin
R' die für die allgemeine Formel (II) genannte Bedeutung hat,
R¹, R² und R³ unabhängig voneinander H, Fluor, Chlor, Brom, Nitro oder Carboxyl bedeuten und
Hal für Fluor, Chlor oder Brom steht.

2. Verfahren nach Anspruch 1, **dadurch gekennzeichnet, dass** die Konzentration der Verbindung der allgemeinen Formel (II) im Reaktionsgemisch bei maximal 7 Gew.-%, bevorzugt bei maximal 6 Gew.-% und insbesondere maximal bei 5 Gew.-% liegt.

3. Verfahren nach Anspruch 1 oder 2, **dadurch gekennzeichnet, dass** in den allgemeinen Formeln (I) und (II) X unabhängig voneinander für einen Sulfonsäure- oder Nitrorest und Y unabhängig voneinander für Fluor oder Chlor steht.

4. Verfahren nach einem oder mehreren der Ansprüche 1-3, **dadurch gekennzeichnet, dass** n für den Wert 0, 1 oder 2 und m für den Wert 0, 1 oder 2 steht, wobei die Summe aus n und m den Wert 1 oder 2 annimmt.

5. Verfahren nach einem oder mehreren der Ansprüche 1-4, **dadurch gekennzeichnet, dass** sich in einer der o-Positionen zu der zu oxidierenden Gruppe -CH₂R ein Wasserstoff-Atom oder ein Fluoratom, bevorzugt ein Wasserstoffatom befindet.

6. Verfahren nach einem oder mehreren der Ansprüche 1-5, **dadurch gekennzeichnet, dass** Nitro-toluol-sulfonsäuren und insbesondere 4-Nitro-toluol-2-sulfonsäure als Verbindung der allgemeinen Formel (II) eingesetzt wird.

7. Verfahren nach einem oder mehreren der Ansprüche 1-6, **dadurch gekennzeichnet, dass** es bei einem Druck von 4 - 15 bar, bevorzugt von 6 - 12 bar und insbesondere von 10-12 bar und bei einer Temperatur von 120 - 200°C und bevorzugt von 140 - 180°C durchgeführt wird.

8. Verfahren nach einem oder mehreren der Ansprüche 1-7, **dadurch gekennzeichnet, dass** eine 10-90%ige, bevorzugt eine 20-65%ige Salpetersäure vorgelegt wird

9. Verfahren nach einem oder mehreren der Ansprüche 1-8, **dadurch gekennzeichnet, dass** das molare Verhältnis der Verbindung der allgemeinen Formel (II) zur Salpetersäure im Bereich von (2-20):1 , bevorzugt im Bereich von (3-10): 1 und besonders bevorzugt im Bereich von (4-8):1 liegt.

10. Verfahren nach einem oder mehreren der Ansprüche 1-9, **dadurch gekennzeichnet, dass** 10-90%, bevorzugt 20-50% der einzusetzenden Salpetersäure vorgelegt und aufgeheizt werden, und dann simultan aber getrennt die Verbindung der allgemeinen Formel (II) und die restliche Salpetersäure in den Reaktor eindosiert werden.

11. Verfahren nach einem oder mehreren der Ansprüche 1-9. **dadurch gekennzeichnet, dass** der Salpetersäure Substanzen hinzugefügt werden, die NOₓ freisetzen oder beinhalten, bevorzugt Alkali- oder Erdalkalinitrite, salpetrige Säure oder konzentrierte rotbraune Salpetersäure.

## Claims

1. Process for preparing nitrobenzoic acids of the general formula (I) 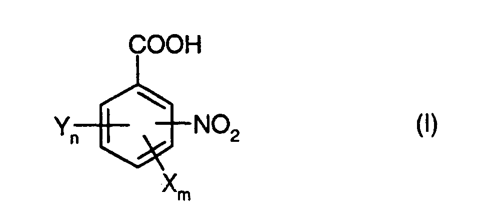 where
each X is independently nitro, cyano, sulphonyl, carboxyl, carbonylphenyl, trifluoromethyl or trichloromethyl,
each Y is independently phenyl, fluorine, chlorine, bromine or iodine and the phenyl radical may in turn be substituted by up to 3 X and/or Y radicals other than hydrogen,
m has the value 0, 1, 2 or 3 and
n has the value 0, 1, 2 or 3,
and the sum of m and n has the value 0, 1, 2, 3 or 4,
from compounds of the general formula (II) 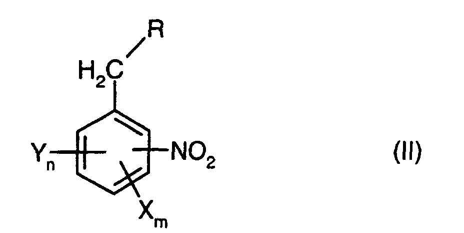 where
X, Y, m, n and also the sum of n and m are each as defined above for the general formula (I) and
R is H or an OR' radical and
R' is H, straight-chain or branched C₁-C₁₀-alkyl or straight-chain or branched C₁-C₁₀-carbonylalkyl or R' is a radical of the general formula (III) 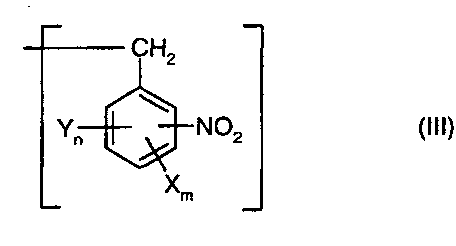
or a radical of the general formula (IV), 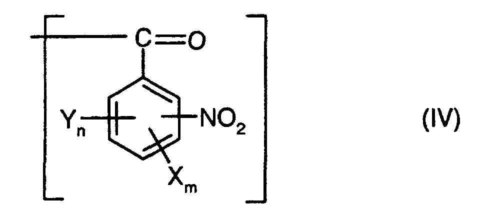 where X, Y, n, m and also the sum of n and m are each as defined for the general formula (I),
**characterized in that** a mixture of nitric acid and water is heated at a pressure in the range of 2-20 bar at temperatures in the range of 100 - 220°C and the compound of the general formula (II) is metered into this heated mixture of nitric acid and water,
although compounds of the general formula (II) exclude 2-halo-6-nitrobenzyl alcohols, esters or ethers of the general formula (V) and 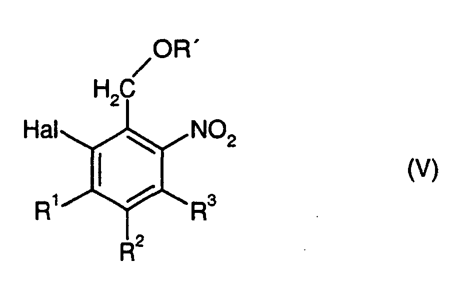 where
R' is as defined for the general formula (II),
R¹, R² and R³ are each independently H, fluorine, chlorine, bromine, nitro or carboxyl and
Hal is fluorine, chlorine or bromine.

2. Process according to Claim 1, **characterized in that** the maximum concentration of the compound of the general formula (II) in the reaction mixture is 7% by weight, preferably 6% by weight and in particular 5% by weight.

3. Process according to Claim 1 or 2, **characterized in that** each X in the general formulae (I) and (II) is independently a sulphonic acid or nitro radical and each Y is independently fluorine or chlorine.

4. Process according to one or more of Claims 1-3, **characterized in that** n has the value 0, 1 or 2 and m has the value 0, 1 or 2, and the sum of n and m takes the value 1 or 2.

5. Process according to one or more of Claims 1-4, **characterized in that** a hydrogen atom or a fluorine atom, preferably a hydrogen atom, is in one of the o-positions to the group -CH₂R to be oxidized.

6. Process according to one or more of Claims 1-5, **characterized in that** the compound of the general formula (II) used is a nitrotoluenesulphonic acid and in particular 4-nitrotoluene-2-sulphonic acid.

7. Process according to one or more of Claims 1-6, **characterized in that** it is carried out at a pressure of 4 - 15 bar, preferably of 6 - 12 bar and in particular of 10 - 12 bar and at a temperature of 120 - 200°C and preferably of 140 - 180°C.

8. Process according to one or more of Claims 1-7, **characterized in that** 10-90%, preferably 20-65%, nitric acid is initially charged.

9. Process according to one or more of Claims 1-8, **characterized in that** the molar ratio of the compound of the general formula (II) to nitric acid is in the range of (2-20):1 , preferably in the range of (3-10):1 and more preferably in the range of (4-8): 1.

10. Process according to one or more of Claims 1-9, **characterized in that** 10-90%, preferably 20-50%, of the nitric acid to be used is initially charged and heated, and then the compound of the general formula (II) and the remaining nitric acid are metered into the reactor simultaneously but separately.

11. Process according to one or more of Claims 1-9, **characterized in that** substances are added to the nitric acid which release or contain NOₓ, preferably alkali metal or alkaline earth metal nitrites, nitrous acid or concentrated red-brown nitric acid.

## Revendications

1. Procédé de production d'acides nitrobenzoïques de formule générale (I) 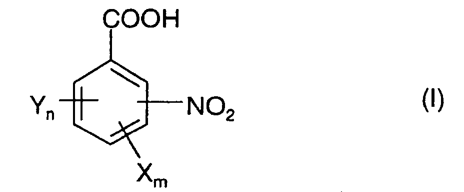 où
les X représentent indépendamment les uns des autres nitro, cyano, sulfo, carboxyle, carbonylphényle, trifluorométhyle ou trichlorométhyle,
les Y représentent, indépendamment les uns des autres, phényle, le fluor, le chlore, le brome ou l'iode et dans lesquels le reste phényle peut lui-même être substitué par jusqu'à 3 restes X et/ou Y différents de l'hydrogène,
m possède la valeur 0, 1, 2 ou 3 et
n possède la valeur 0, 1, 2 ou 3,
et la somme de m et n possède la valeur 0, 1, 2, 3 ou 4,
à partir de composés de formule générale (II) 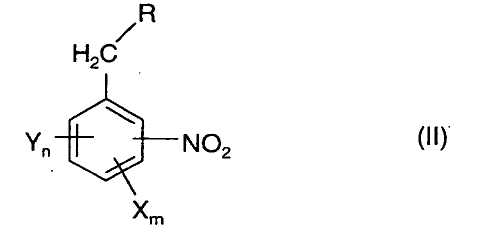 où
X, Y, m, n et la somme de n et m ont les significations citées pour la formule générale (I) et
R représente H ou un reste OR' et
R' représente H, alkyle en C₁-C₁₀ linéaire ou ramifié ou carbonylalkyle en C₁-C₁₀ linéaire ou ramifié ou bien R' représente un reste de formule générale (III) 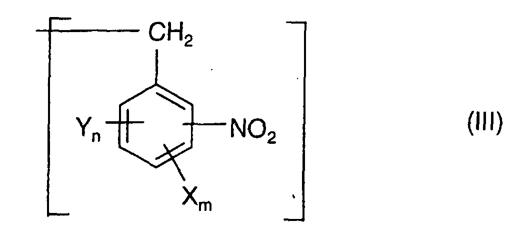
ou un reste de formule générale (IV) 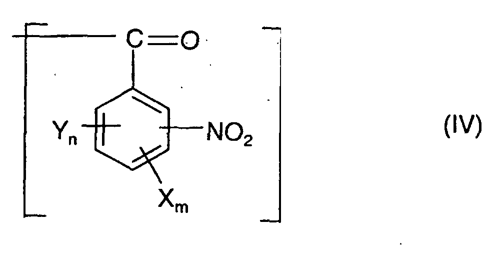 où X, Y, n, m et la somme de n et m possèdent dans chaque cas les significations citées pour la formule générale (I),
**caractérisé en ce qu'**un mélange d'acide nitrique et d'eau est chauffé à une pression dans le domaine de 2-20 bars à des températures dans le domaine de 100-220°C et le composé de formule générale (II) est introduit dans ce mélange chauffé d'acide nitrique et d'eau,
où toutefois, comme composés de formule générale (II), les alcools, esters ou éthers 2-halogéno-6-nitrobenzyliques de formule générale (V) sont exclus, 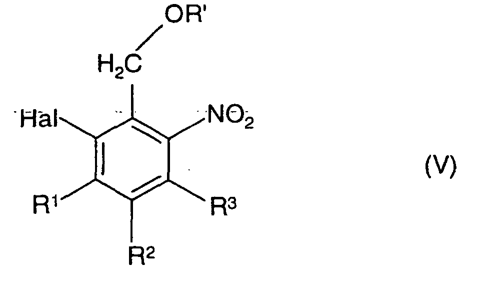 où
R' a la signification citée pour la formule générale (II),
R¹, R² et R³ représentent indépendamment l'un de l'autre H, le fluor, le chlore, le brome, nitro ou carboxyle et
Hal représente le fluor, le chlore ou le brome.

2. Procédé selon la revendication 1, **caractérisé en ce que** la concentration du composé de formule générale (II) dans le mélange réactionnel est située au maximum à 7 % en masse, de préférence au maximum à 6 % en masse et en particulier au maximum à 5 % en masse.

3. Procédé selon la revendication 1 ou 2, **caractérisé en ce que**, dans les formules générales (I) et (II), les X représentent indépendamment les uns des autres un reste acide sulfonique ou nitro et les Y représentent indépendamment les uns des autres le fluor ou le chlore.

4. Procédé selon une ou plusieurs des revendications 1-3, **caractérisé en ce que** n représente la valeur 0, 1 ou 2 et m représente la valeur 0, 1 ou 2, la somme de n et m prenant la valeur 1 ou 2.

5. Procédé selon une ou plusieurs des revendications 1-4, **caractérisé en ce qu'**un atome d'hydrogène ou un atome de fluor, de préférence un atome d'hydrogène, se trouve dans l'une des positions o par rapport au groupe à oxyder -CH₂R.

6. Procédé selon une ou plusieurs des revendications 1-5, **caractérisé en ce que** des acides nitrotoluènesulfoniques et en particulier l'acide 4-nitrotoluène-2-sulfonique sont utilisés comme composé de formule générale (II).

7. Procédé selon une ou plusieurs des revendications 1-6, **caractérisé en ce qu'**il est mis en oeuvre à une pression de 4-15 bars, de préférence de 6-12 bars et en particulier de 10-12 bars et à une température de 120-200°C et de préférence de 140-180°C.

8. Procédé selon une ou plusieurs des revendications 1-7, **caractérisé en ce qu'**un acide nitrique à 10-90 %, de préférence à 20-65 %, est disposé au préalable.

9. Procédé selon une ou plusieurs des revendications 1-8, **caractérisé en ce que** le rapport molaire du composé de formule générale (II) à l'acide nitrique est situé dans le domaine de (2-20):1, de préférence dans le domaine de (3-10):1 et de manière particulièrement préférée dans le domaine de (4-8):1.

10. Procédé selon une ou plusieurs des revendications 1-9, **caractérisé en ce que** 10-90 %, de préférence 20-50 %, de l'acide nitrique à utiliser sont disposés au préalable et chauffés, puis le composé de formule générale (II) et l'acide nitrique restant sont introduits dans le réacteur simultanément mais séparément.

11. Procédé selon une ou plusieurs des revendications 1-9, **caractérisé en ce que** des substances qui libèrent ou contiennent NOₓ, de préférence des nitrites alcalins ou alcalino-terreux, l'acide nitreux ou l'acide nitrique brun-rouge concentré, sont ajoutées à l'acide nitrique.
